# EUROPEAN PATENT APPLICATION

(11) **EP 4 082 545 A1**
(43) Date of publication of application: **02.11.2022**
(21) Application number: 21305543.7
(22) Date of filing: 27.04.2021
(51) Int. Cl.: A61K 31/496, A61K 31/665, A61K 35/74, A61K 45/06, A61P 31/04

(54) **COMBINATION PRODUCT AND METHODS FOR PREVENTING THE EMERGENCE OF ANTIBIOTIC-RESISTANT BACTERIA UNDER ANTIBIOTIC TREATMENT**

(71) Applicant: Diotheris, 78180 Montigny-le-Bretonneux (FR); Université de Versailles Saint-Quentin-en-Yvelines, 78035 Versailles Cédex (FR); ASSISTANCE PUBLIQUE, HOPITAUX DE PARIS, 75004 Paris (FR)
(72) Inventor: GAILLARD, Jean-Louis, 92130 ISSY LES MOULINEAUX (FR); ROTTMAN, Martin, 78170 LA CELLE SAINT-CLOUD (FR); BAERISWYL, Simon, 75007 PARIS (FR); RIBEIRO, Cassie, 78530 BUC (FR)
(74) Representative: Regimbeau

(57) **Abstract**

The present invention relates to a combination product comprising an antibiotic and at least one non-pathogenic microorganism for simultaneous, separate, or sequential use in preventing emergence of resistance to the antibiotic when treating a colonization and/or an infection caused by a bacterium that is susceptible to said antibiotic, wherein said at least one non-pathogenic microorganism is resistant to said antibiotic.

## Description

Antimicrobial resistance (AMR) is a global threat that is jeopardizing the benefits of 80 years of progress, made since the discovery of antimicrobial therapy. Indeed, while the five most common hospital acquired microorganisms currently cause the death of an estimated 25,000 people each year in the European Union, AMR is projected to cause 10 million deaths by 2050 with a cost of $100tn (Bulletin of the World Health Organization, 2016). Furthermore, while resistance to antimicrobial agents is escalating, the discovery of new classes of antimicrobials is almost at a standstill, with the emergence of new resistance determinants far outpacing the release of novel agents. Thus, emergence of resistance in microorganisms undergoing antimicrobial (e.g., antibiotic) therapy is a major issue.

De novo mutation is a major contributor to the development of AMR in bacteria, and notably depends on the generation of genetic changes in antibiotic targets or in the pathways governing antibiotic neutralization. These changes occur during the course of treatment and allow bacteria to survive the antibiotic concentrations achieved in the tissue at a site of colonization and/or infection. This resistance can occur regardless of the antibiotic route of delivery (i.e., per os, IV, IM, topical, etc.). The emergence of mutational resistance is best known for specific drug/bacteria combinations, which include several of the notorious ESKAPE microorganisms (*Enterobacter, Staphylococcus aureus, Klebsiella, Acinetobacter, Pseudomonas aeruginosa, Enterococcus*), identified by the World Health Organization as being the greatest threats to human health given the increasing prevalence of clones that are resistant to a large number of antibiotics, e.g., rifampicin, fosfomycin, mupirocin for *Staphylococcus aureus,* quinolones for all organisms, beta-lactams for *Enterobacter* sp, carbapenems for *P. aeruginosa,* although it is also relevant to several other families of antimicrobials and bacterial species.

This phenomenon of *de novo* mutational resistance arising during antimicrobial treatment is of foremost concern in cases in which bacteria chronically colonize or infect humans (e.g., nasal colonization with *Staphylococcus aureus, Cutibacterium acnes* acting as an inflammatory trigger of acne, *Helicobacter pylori-associated* chronic gastritis, tuberculosis) and has grave consequences for both the individual patient and for the community overall.

From the patient's perspective, resistance can result in treatment failure, as a result of either primary failure or relapse with bacteria having newly acquired resistance, and is associated with significant morbidity and possible mortality.

From a community perspective, acquisition of resistance will result in prolonged length of hospital stays, increased usage of second line antimicrobials, and a deterioration of a clinical unit's ecology with the risk of dissemination of the resistant microorganism to other patients. Furthermore, consecutive treatment failures with different antibiotics may ultimately result in the emergence of multi-drug resistant (MDR) microorganisms. On a global scale, mutational resistance has, for example, caused the selection and dissemination of both MDR and extensively drug-resistant (XDR) *Mycobacterium tuberculosis,* which represents a major public health threat.

In view of limiting mutational resistance, two major strategies have been developed: the use of combinations of two or more antibiotics from different classes and the use of shortened antibiotic treatment regimens. These strategies are based on the fact that the emergence of a resistance-causing mutation is a stochastic event governed by size of the bacterial inoculum, the frequency of the event for each drug-bacteria combination, and the duration of exposure to the antibiotic, and are discussed below.

Firstly, the use of a combination of two antibiotics decreases the probability of mutational resistance, given that antibiotics of different classes have different pathways leading to resistance. Thus, emergence of a microorganism resistant to two different classes of antibiotic requires the simultaneous mutation of both pathways in the same microorganism. However, different bioavailability profiles of each of the antibiotics can result in the intermittent presence of both agents, providing a window for the selection of a mutant to a first agent in the absence of an inhibitory concentration of said agent. Once this resistance is acquired, there is no safeguard preventing the emergence of resistance to the second agent. In addition, when there is a risk of preexisting mutations due to previous therapeutic failures, the combination of three or more agents becomes necessary. While phenotypic and genotypic examination of the colonizing and/or infecting microorganism can be performed to detect resistance-creating mutations and adequately choose the combined agents, this delays therapy and increases its cost and complexity. Moreover, the safety provided by a combination of multiple antibiotics is relative and requires the skillful handling of the agents along with a high level of patient compliance to maximize effectiveness. In addition, the use of multiple antibiotics is associated with increased toxicity and a multiplication of possible side effects, higher price, and ecological pressure in environments that are not specifically targeted by the combination.

The use of shorter treatment regimens can also reduce the emergence of mutational resistance, as the probability of selecting for a resistance mutation is reduced over a lower number of replicative events. For instance, the use of mupirocin in nasal decolonization of S. *aureus* is limited to a 5-day treatment course, as a longer duration of administration selects for resistant mutants. However, the effect of shortened regimens on colonization and/or infection may remain insufficient. In an effort to enhance the treatment effect, repeated courses of the 5-day regimen can be undertaken provided that a three-week washout period is respected. However, this strategy requires months-long therapy and long-term patient compliance, with uncertain efficacy.

Several additional approaches for preventing the emergence mutational resistance to antibiotics have been tested in the past or are currently under development, including vaccination against bacterial pathogens, bacteriophage therapy, and, more recently, microbiome therapy (especially against intestinal bacterial pathogens) through fecal matter transplant (FMT) or administration of selected live bacteria/bacterial cocktails. However, each of these strategies is subject to major limitations. In particular, vaccine development is lengthy with high clinical development costs and ultimately requires a large proportion of the population to be vaccinated to achieve herd immunity. Bacteriophage therapy faces manufacturing difficulties with combinations of bacteriophages required to prevent emergence and suffers from a relative lack of controlled good clinical practice trials. FMT also remains challenging for technical and regulatory reasons. It notably requires significant donor screening, and has suffered from public aversion to the concept. More recently, the formulation of a bacterial cocktail capable of conferring the benefits of fecal transplant without the risk of communicating bacterial or viral pathogens as part of the transferred flora (e.g., "artificial stool") has been explored. This approach is promising in the control of C. *difficile* disease but has not shown efficacy in the decolonization of multi-drug resistant Enterobacteriaceae. Furthermore, projects targeting other pathogens, especially non-intestinal pathogens, are scarce and in the early stages of development, with any commercialization remaining at least several years away.

As current strategies are unsatisfactory, there remains a need for novel products and methods which are able to prevent emergence of antibiotic resistance in bacteria responsible for undesired colonization and/or infection. In particular, such a product should allow for the use of combination therapy using multiple antibiotics to be avoided, thus reducing the negative impact on microbial communities established in the host.

In addition, such a product should be easy to produce, and have relatively few components, in notable contrast to "artificial stool" which seeks to reproduce complex fecal material.

The present invention responds to these and other needs. Specifically, the present invention is directed to a combination product comprising an antibiotic and at least one non-pathogenic microorganism for simultaneous, separate, or sequential use in preventing emergence of resistance to the antibiotic when treating colonization and/or infection caused by a bacterium that is susceptible to said antibiotic, and wherein said at least one non-pathogenic microorganism is resistant to said antibiotic. The combination product based on the administration of both an antibiotic and at least one non-pathogenic microorganism advantageously prevents emergence of resistance in the treated bacteria. Advantageously, the combination product can be administered for longer periods of time than an antibiotic alone. Advantageously, the combination product allows for antibiotics which are known to be associated with rapid development of antibiotic resistance in bacteria, and which otherwise may not be considered as a first-line or desired treatment, to be used in treating bacterial colonization and/or infection. Thus, the range of antibiotics available for treating a given bacterial colonization and/or infection may be expanded. As a result of the limited number of components comprised in the combination product, it is also relatively inexpensive.

Preferably, the non-pathogenic microorganism is naturally resistant to the antibiotic, is selected in the presence of an antibiotic, or is genetically modified to be resistant to the antibiotic.

Preferably, resistance to the antibiotic is due to at least one nucleotide mutation in a gene or the introduction of a non-transferable gene.

Preferably, at least 10³ CFUs of the non-pathogenic microorganism is administered per dose, more preferably 10³ to 10⁹ CFUs per dose.

Preferably, the non-pathogenic microorganism is administered on the same day as, but before, the antibiotic.

Preferably, the antibiotic is associated with the development of mutational resistance in bacteria when used alone.

Preferably, the antibiotic is selected from fosfomycin and rifamycins, such as rifampicin.

Preferably, the non-pathogenic microorganism is a bacterium or yeast, preferably a bacterium.

Preferably, the non-pathogenic microorganism belongs to the *Corynebacterium* or *Staphylococcus* genus. Preferably the non-pathogenic microorganism is *Corynebacterium aurimucosum, Corynebacterium philbarense, Corynebacterium afermentans,* or *Staphylococcus epidermidis.*

Preferably, the non-pathogenic microorganism is *Corynebacterium aurimucosum, Corynebacterium philbarense,* or *Corynebacterium afermentans* and the antibiotic is fosfomycin or rifampicin.

Preferably, the combination product provided herein further comprises at least one pharmaceutically acceptable excipient.

Preferably, the non-pathogenic microorganism is administered topically or orally, more preferably topically.

Preferably, the antibiotic is administered topically, orally, or parenterally, more preferably topically.

### DETAILED DESCRIPTION

Before describing the invention in further detail, it should be noted that the terms "a", "an" and "the" as used herein are used in the sense that they mean "at least one", "at least a first", "one or more" or "a plurality" of the referenced compounds or steps, unless the context dictates otherwise. As an example, reference to "the non-pathogenic microorganism" therefore also includes two or more non-pathogenic microorganisms.

The term "and/or" as used herein includes the meaning of "and", "or" and "all or any other combination of the elements connected by said term". The terms "comprising", "having", "including", and "containing" (and any form of said terms, such as e.g., "contains" or "contain") are open-ended and do not exclude additional, unrecited elements or method steps. In contrast, the term "consisting of" as used herein excludes any other components (beyond trace levels) or steps.

A combination product comprising an antibiotic and at least one non-pathogenic microorganism that is resistant to said antibiotic is provided herein. Said combination product prevents emergence of resistance to an antibiotic when treating colonization and/or an infection caused by a bacterium, wherein the bacterium is susceptible to said antibiotic. The invention thus more particularly provides a combination product comprising an antibiotic and at least one non-pathogenic microorganism for simultaneous, separate, or sequential use in preventing emergence of resistance to the antibiotic when treating a colonization and/or an infection caused by a bacterium that is susceptible to said antibiotic, wherein said at least one non-pathogenic microorganism is resistant to said antibiotic.

As used herein, the terms "combination product" or "composition" refer to a combination of products that are provided either in a single dosage unit form or as a kit of parts for the combined administration of the products. The products of the combination (i.e., the antibiotic and the at least one non-pathogenic microorganism) may be used simultaneously, separately, or sequentially.

By "simultaneous" use is meant herein an administration of the antibiotic and the at least one non-pathogenic microorganism by the same route (i.e., together) and at the same time. Simultaneous administration refers to administration of an admixture (whether a true mixture, a suspension, an emulsion, or other physical combination). As such, the combination may be already provided as an admixture, or as separate containers of the antibiotic and the at least one non-pathogenic microorganism that are combined together into an admixture prior to administration.

By "separate" use is meant herein an administration of the antibiotic and the at least one non-pathogenic microorganism separately of each other at the same time or at substantially the same time, by different routes. By "substantially the same time" is meant that no intentional lapse of time occurs between administration of the two products. Any temporal delay may e.g., correspond to the amount of time necessary to separately administer each product via each route of administration.

By "sequential" use is meant herein an administration of the antibiotic and the at least one non-pathogenic microorganism separately of each other and at different times, by the same or different routes. More particularly, the complete administration of one of the products is carried out before administration of the other product begins. Frequency of administration may furthermore differ between the two products. For example, the antibiotic may be administered multiple times per day while the non-pathogenic microorganism is administered only once per day, or vice versa. Preferably, the non-pathogenic microorganism is administered on the same day as, but before, the antibiotic. Alternatively, the antibiotic may be administered on the same day as, but before, the non-pathogenic microorganism. In cases where the antibiotic is administered two or more times in one day, the non-pathogenic microorganism may be administered before and/or after the antibiotic or vice versa. For patient compliance, it is preferable, when possible, to limit the frequency of administration of the of the antibiotic and/or the non-pathogenic microorganism to three times per day, more preferably to twice per day, even more preferably to once per day, most preferably to less than once per day (e.g., every two days). When the two products are administered sequentially, they are nevertheless administered with a temporal proximity that is sufficient to ensure that the advantageous effect of the combined products (i.e., the prevention of the emergence of resistance to the antibiotic in the bacteria being treated) indeed occurs.

The term "antibiotic" as used herein refers to any substance, compound or chemical that is used in the treatment of a bacterial colonization and/or infection. An antibiotic may either kill or inhibit the growth of bacteria. Antibiotics that may be used in the present invention include e.g., penicillins, cephalosporins, carbapenems, aminoglycosides, quinolones, antibiotics of the macrolides-lincosamides-streptogramins (MLS) family, lipopeptides, glycopeptides, glycolipopeptides, oxazolidinones, polymyxins, sulfonamides, pleuromutilins, ansamycins, lincomycins, and tetracyclines. Preferred antibiotics include: amikacin, amoxicillin, amoxicillin/K clavulanate, ampicillin, ampicillin/sulbactam, azithromycin, aztreonam, bacitracin, cefaclor, cefazolin, cefepime, cefotaxime, cefotaxime/K clavulanate, cefoxitin, ceftazidime, ceftazidime/K clavulanate, ceftazidime/avibactam, ceftdinir, ceftolozane/tazobactam, ceftriaxone, cefuroxime, chloramphenicol, chlortetracycline, clindamycin, ciprofloxacin, dalbavancin, daptomycin, demeclocycline, dicloxacillin, doripenem, doxycycline, eravacycline, ertapenem, erythromycin, fosfomycin, fusidic acid, gentamicin, gramicidin, imipenem, imipenem/cilastatin, imipenem/cilastatin/relebactam levofloxacin, linezolid, lincomyin, mafenide, meropenem, minocycline, moxifloxacin, mupirocin, neomycin, nitrofurantoin, omadacycline, oritavancin, oxacillin, oxytetracycline, ozenoxacin, penicillin, piperacillin, piperacillin/tazobactam, polymyxin b, pramoxine, quinupristin-dalfopristin, retapamulin, rifampicin, staphylomycin, streptomycin, sulfacetamide, sulfadiazine, sulfamethoxazole and trimethoprim, tedizolid, teicoplanin, telavancin, tetracycline, ticarcillin/K clavulanate, tigecycline, tobramycin, trimethoprim/sulfamethoxazole, vancomycin, virginiamycin, tedizolid, or a combination of two or more thereof.

Particularly preferred antibiotics are those that are administered topically, orally, or parenterally, more preferably chosen among the antibiotics listed above which are topically, orally or parenterally administered, even more preferably chosen among the antibiotics listed above which are topically administered. According to a particularly preferred embodiment, the topical antibiotic is chosen among bacitracin, clindamycin, daptomycin, mupirocin, erythromycin, sulfacetamide (e.g., sulfacetamide sodium, sulfacetamide sodium-sulfur), chlortetracycline, demeclocycline, fusidic acid, neomycin, ozenoxacin, polymyxin B, pramoxine, sulfadiazine (e.g. silver sulfadiazine), retapamulin, gentamicin, gramicidin, mafenide, virginiamycin, chloramphenicol, oxytetracycline, linezolid, tedizolid and combinations of two or more thereof (e.g., bacitracin/polymyxin b, bacitracin/neomycin/polymyxin b, polymyxin B sulfate-gramicidin, bacitracin/neomycin/polymyxin b/pramoxine, neomycin/polymyxin b/pramoxine).

An antibiotic that is preferably incorporated into the combination product of the present invention is one that is associated with the development of mutational resistance in a bacterium colonizing or causing an infection when said antibiotic is used alone (i.e., with no other treatment). In other words, resistance emerges in the bacteria being treated by the antibiotic. The term "mutational resistance" as used herein refers to an acquired resistance that arises when a mutation occurs in the bacterial genome, e.g., within a gene that codes a protein that is targeted by the antibiotic. The mutation may be e.g., a substitution, a deletion, or an insertion of one or more nucleotides in the genome. The mutation may arise spontaneously as a result of random genetic mutations.

The antibiotic is preferably provided at a concentration that is sufficient to be therapeutically effective when treating bacteria that are considered to be susceptible at a given body site (e.g., a site of undesired bacterial colonization and/or infection). Bacteria are considered to be "susceptible" or "sensitive" to an antibiotic when their growth is inhibited or when they are killed by the usually achievable concentrations of the antibiotic when the recommended dosage is used. Occasionally, bacteria may have "intermediate susceptibility." In this case, at the minimum inhibitory concentration (MIC) of an antibiotic, which approaches usually attainable blood and tissue levels, growth of a microorganism is higher than for susceptible bacteria. However, an antibiotic may nevertheless be effective in treating bacteria having intermediate susceptibility at sites where the antibiotic is physiologically concentrated or when a higher-than-normal dosage can be used.

In some cases, the emergence of resistance may be associated with exposure of a sensitive bacteria to the antibiotic for a particular length of time or at a particular concentration. In some cases, the bacterium causing a colonization and/or infection has a high mutation rate (e.g., hypermutable bacterial strains), such that antibiotic resistance quickly emerges when the bacterium is exposed to an antibiotic, even though it was initially susceptible. In notable contrast, when an antibiotic is comprised within the combination product of the invention rather than used alone, emergence of antibiotic resistant mutants can be prevented.

Preferably, the antibiotic is selected from the ansamycin family, preferably from among rifamycins, or from phosphonic acid derivatives, aminoacyl-tRNA synthetase inhibitors, or quinolones. Preferably, the rifamycin antibiotic is selected from among rifampicin (also referred to as rifampin), rifabutin, rifapentine, rifalazil, and rifaximin. Preferably, the antibiotic is selected from phosphonic acid derivatives, such as fosfomycin, fosmidomycin, and alafosfalin. The aminoacyl-tRNA synthetase inhibitor is preferably an isoleucyl-tRNA synthetase inhibitor, more preferably mupirocin. The quinolone is preferably a fluoroquinolone. The quinolone is preferably selected from among norfloxacin, lomefloxacin, ofloxacin, ciprofloxacin, fleroxacin, nadifloxacin, pefloxacin, rufloxacin, levofloxacin, moxifloxacin, balofloxacin, grepafloxacin, pazufloxacin, sparfloxacin, temafloxacin, clinafloxacin, gatifloxacin, sitafloxacin, prulifloxacin, besifloxacin, delafloxacin, danofloxacin, difloxacin, enrofloxacin, ibafloxacin, marbofloxacin, orbifloxacin, and sarafloxacin. More preferably, the antibiotic is fosfomycin or a rifamycin, even more preferably rifampicin. Preferably, when the combination product comprises fosfomycin, said antibiotic is provided at 3 g of fosfomycin per dose for oral administration or at a concentration of 3% for topical application. Preferably, when the combination product comprises rifampicin, said antibiotic is provided at 50, 120, 150, 300 or 600 mg of rifampicin per dose for oral administration, 1% for topical administration, or 600 mg of rifampicin for intravenous administration. In some cases, the antibiotic concentration in the combination product is lower than the usual therapeutic concentration, in particular in the case of topical administration of the product. Indeed, topical antibiotics are often administered at concentrations that are much higher than necessary to achieve a therapeutic effect.

The term "dose" is defined herein as the amount of the respective products present in the combination that is administered at a given time. The combination product may be administered as a single dose. Alternatively, said product may be administered as in multiple doses over a period of time (e.g., days, weeks, or months). In particular, administration of the combination product may be repeated as appropriate to successfully prevent emergence of antibiotic resistance in bacteria while concomitantly treating a bacterial infection and/or colonization. A "dose" may be expressed herein in terms of weight, volume, concentration, as a function of body weight or body surface area, or as CFUs of either product of the combination or of the combination product, as appropriate. Dosage information is provided for each of the antibiotic and the at least one the non-pathogenic microorganism. Antibiotic dosage is generally expressed in weight or concentration, while non-pathogenic microorganism dosage is generally expressed in CFUs. As a non-limiting example, fosfomycin may be orally administered at a dose of 3g in adults or in children under 12 years of age at a total daily dose (which may be divided into two or more individual doses) of 200-400 mg/kg.

The combination product of the present invention advantageously prevents emergence of resistance when treating a colonization and/or infection caused by bacteria that are susceptible to the antibiotic. The expression "preventing emergence of resistance" as used herein notably refers to a reduction or inhibition of the appearance of antibiotic-resistance mutants of a bacteria colonizing and/or causing infection at a body site. Thus, emergence of resistance is advantageously slowed, or, more preferably, completely inhibited by the combination product.

The expressions "colonization caused by a bacterium," "colonization by a bacterium," and "bacterial colonization," refer interchangeably herein to the undesired presence of at least one bacterial species or strain at a given body site (e.g., on the skin, on a mucosal surface). While microorganisms naturally colonize various sites of the human body, undesired colonization by a bacterium (i.e., by a "colonizing bacterium") as provided herein may notably arise when said bacterium colonizes a site where it is not normally present as part of the natural flora or when said bacterium is naturally present but is undesirable as it is associated with e.g., an increased risk of an infection. In some cases, said bacterium may be present in a higher quantity than is desired (e.g., at a higher quantity than is present at a healthy site or in a healthy subject). In particular, colonization by a bacterium at a particular site (or at high levels) may increase the risk that a subject will develop an infection caused by said bacterium either at said site or at a different site (e.g., during surgery or dialysis). As a particular example, colonization by *S*. *aureus* is associated with an increased risk of endogenous *S*. *aureus* infection, in particular in surgical and dialysis patients. Thus, it is advantageous to treat colonization of *S*. *aureus* in a subject, in particular in the nares, prior to performing surgery. The "colonization caused by a bacterium" as used herein is to be distinguished from colonization by the non-pathogenic microorganism that may result from treatment of undesired bacterial colonization and/or infection with the combination product.

An "infection caused by a bacterium" or a "bacterial infection" as used herein refers to any pathogenesis of a disease or condition that is present in a host and in which bacteria are involved or associated. An "infection caused by a bacterium" may notably result from bacterial colonization of a body site.

Colonization and/or infection caused by a bacterium may notably occur in a patient with comorbidity, as a result of hormonal changes, wounding, malnutrition, stress, and/or drug regimens which may notably suppress inflammatory and/or immune responses. Preferably, the colonization and/or infection involves only a single bacterial species or strain. However, in some cases, colonization and/or infection may result from two, three, four, or more bacterial species or bacterial strains. In cases where multiple bacterial species or strains are associated with the colonization and/or infection, said species or strains are preferably all susceptible to the antibiotic present in the combination product prior to treatment.

The bacterium causing a colonization and/or an infection may be gram negative, gram positive, or an atypical bacterium. It may be anaerobic, microaerobic, or aerobic. Preferably, the bacteria are pathogenic bacteria (i.e., capable of causing disease or illness in a human subject). The bacteria may be a facultative or opportunistic pathogen. For example, a bacterium may be present as a non-pathogenic at a first site on a host and become pathogenic only following e.g., a physiological change at the first site or when introduced at a second site which it does not normally colonize. This is notably the case for *S*. *aureus,* which frequently colonizes the nares and/or the skin of human subjects. Indeed, *S*. *aureus* is also responsible for a wide range of pathologies including skin or soft tissue infection (SSTI), wound infection, bacteremia, endocarditis, pneumonia, osteomyelitis, toxic shock syndrome, infective endocarditis, folliculitis, furuncle, carbuncle, impetigo, bullous impetigo, cellulitis, botryomyosis, scalded skin syndrome, central nervous system infection, infective and inflammatory eye disease, osteomyelitis or other infections of joints or bones, respiratory tract infection, urinary tract infection, septic arthritis, septicemia, or gangrene. Such pathologies may notably result from a breach in the skin or mucus membrane, invasive surgery, presence of a foreign device or implant in the subject, immunological deficiencies in the human host, etc. This is also the case for *C. acnes,* which is part of healthy skin microflora, but which in some cases is associated with inflammatory skin diseases such as acne, spondylodiscitis, or implant infections.

As a non-limiting example, the bacteria causing a colonization and/or an infection may belong to the family of Staphylococcaceae, Streptococcaceae, Enterococcaceae, Enterobacteriaceae, Clostridiaceae, Peptostreptococcaceae, Propionibacteriaceae, Helicobacteraceae, etc. Preferably, bacteria belong to one of the following genera: *Staphylococcus* (e.g., *S. aureus,* as mentioned above), *Streptococcus* (e.g., *S. pyogenes, S. pneumoniae, S agalactiae*), *Enterococcus* (e.g., *E. faecalis* or *E. faecium), Bacillus* (e.g., *B. anthracis*), *Listeria* (e.g., *Listeria monocytogenes), Clostridioides* (e.g., *C. difficile), Clostridium (e.g., C. perfringens, C. tetanus, C. botulinum), Neisseria* (e.g., *N. meningitidis, N. gonorrhoeae), E. coli, Shigella* species, *Salmonella* species, *Yersinia* (e.g., *Y. pestis, Y. pseudotuberculosis, Y. enterocolitica*), *Vibrio cholerae, Campylobacter* (e.g., *C. jejuni, C. fetus), Helicobacter pylori, Pseudomonas* (e.g., *P. aeruginosa, P. mallei*), *Haemophilus influenzae, Bordetella pertussis, Mycoplasma pneumoniae, Ureaplasma urealyticum, Legionella pneumophila*, *Treponema pallidum*, *Leptospira interrogans, Borrelia burgdoferi, Cutibacterium (e.g., C. acnes*), *Mycobacterium* (e.g., M. *tuberculosis, M. leprae*), *Actinomyces* species, *Chlamydia* (e.g., *C. psittaci, C. trachomatis, C. pneumoniae), Rickettsia* (e.g., *R. ricketsii, R. prowazekii, R. akari), Brucella* (e.g., *B. abortus*, *B. melitensis, B. suis), Proteus mirabilis*, *Serratia marcescens, Enterobacter clocae*, *Acetinobacter anitratus*, *Klebsiella pneumonia* and *Francisella tularensis.* More preferably, the bacterium causing a colonization and/or an infection is *S. aureus.*

Said bacteria causing a colonization and/or an infection may be resistant to one or more antibiotics or other antibacterial agents, so long as it is susceptible to the antibiotic present in the combination product provided in the present invention. When said bacteria causing a colonization and/or an infection is *S. aureus,* said *S. aureus* may thus notably be a methicillin-resistant *S. aureus* (MRSA) or a methicillin-susceptible *S. aureus* (MSSA).

The term "non-pathogenic microorganism" as used herein refers to a microorganism that can occupy or colonize a site on or in a host (e.g., a human host), but that does not cause disease at the selected site. The non-pathogenic microorganism may be a gram-negative, gram-positive, or atypical bacteria. The non-pathogenic microorganism may be anaerobic, microaerobic, or aerobic. The non-pathogenic microorganism may belong to the same family or genus as the bacteria causing a colonization and/or an infection. The non-pathogenic microorganism may have a symbiotic or mutualistic relationship with its host. The site colonized by the non-pathogenic microorganism may notably be the epidermis (i.e., skin), mucus membranes, or gastrointestinal tract, more particularly mucus membranes such as the nares or sinuses, tracheal, gingival, pharyngeal, bronchial, gastric, duodenal, ileal, jejunal, colonic, or vaginal mucosal surfaces, on the axilla, groin, inguinal and perirectal regions, or elsewhere on the skin or mucus membranes. A "mucus membrane" or a "mucosal surface" refers to a tissue layer found lining various tubular cavities of the body such as the oropharynx, small intestine, large intestine, rectum, penis, vagina, mouth, uterus, etc., such as those listed above. This membrane is typically colonized by a variety of microorganisms even when the host is healthy. Among the non-pathogenic microorganisms which can be used as part of the combination product of the present invention are those which naturally inhabit a site that is subject to colonization and/or infection caused by bacteria or those which have been manipulated (e.g., adapted) so that they can colonize said site. Exemplary methods for adapting non-pathogenic microorganisms are discussed below. Preferably, the non-pathogenic microorganism is one that naturally inhabits the site. The non-pathogenic microorganism is preferably a commensal microorganism. In some cases, the non-pathogenic microorganism may be a selected or genetically engineered version of a species or strain that naturally inhabits the site which is being colonized. A non-pathogenic microorganism that can "colonize" a site is one that can compete with the preexisting microflora and/or the bacteria associated with an undesired colonization and/or infection, and take up residence at the site. Colonization by the non-pathogenic microorganism may be temporary (e.g., lasting for the duration of treatment of a bacterial colonization and/or infection with the combination product or persisting beyond the duration of treatment of a bacterial colonization and/or infection with the combination product for a certain amount of time, such as several days, weeks, months, or years) or permanently. Said non-pathogenic microorganism may be a bacterium, yeast or fungus, more preferably a bacterium or yeast, even more preferably a bacterium. Said non-pathogenic microorganism may more particularly be a probiotic or a live biotherapeutic. In some cases, the non-pathogenic microorganism may be a modified version of a strain that has been previously administered to humans as a probiotic or a live biotherapeutic and which is known to occupy or colonize a site and to be non-pathogenic.

The at least one non-pathogenic microorganism is resistant to the antibiotic that is comprised in the combination product along with said microorganism. As used herein, the term "resistance" indicates that growth of a microorganism is not inhibited by the usually achievable concentrations of the antibiotic when the recommended dosage is used and when administered according to a normal dosage schedule. Antibiotic resistance as used herein with regard to the non-pathogenic microorganism more particularly means that the non-pathogenic microorganism is not subject to growth inhibition or death as a result of exposure to the antibiotic at the concentration that would otherwise inhibit the growth or kill a susceptible bacterium (i.e., one which has no resistance). As a result of the antibiotic resistance of the non-pathogenic microorganism, the presence of the antibiotic in the combination product does not negatively affect the growth of the non-pathogenic microorganism.

In one aspect, the microorganism is naturally resistant to the antibiotic. By "naturally resistant" is meant herein that the resistance element is determined by bacterial chromosome genes, arose without human intervention, and is passed from generation to generation. Resistance is thus stable. Alternatively, the microorganism is selected in the presence of an antibiotic or is genetically modified to be resistant to the antibiotic. As an example, selection of a non-pathogenic microorganism that is resistant to the antibiotic may occur when the non-pathogenic microorganism is submitted to appropriate exposure to the antibiotic, or by gene mutation (e.g., site directed mutagenesis), preferably in a chromosomal gene. Preferably, resistance of the non-pathogenic microorganism to the antibiotic is due to the presence of at least one nucleotide mutation in a gene or to the presence of a gene that counteracts the effect of the antibiotic. Antibiotic resistance may alternatively be acquired by horizontal gene transfer, e.g., by transformation, transduction, conjugation, from a bacteriophage, etc. or via direct mutation. Preferably, resistance to the antibiotic in the non-pathogenic microorganism cannot be transferred to any other microorganism, including the bacteria being treated. Preferably, resistance to the antibiotic is due to the introduction of a gene, preferably a non-transferable gene. A "non-transferable gene" as used herein refers to a gene that notably cannot be transferred to another microorganism by conjugation.

The non-pathogenic microorganism may inhibit the growth of the colonizing bacteria or bacteria causing the infection. The non-pathogenic microorganism may inhibit or neutralize the virulence of the bacteria causing the infection. As a particular example, the non-pathogenic microorganism may inhibit growth of the bacteria by outcompeting it at the colonization site as a result of higher growth rate or a greater affinity to the site. As another example, the non-pathogenic microorganism may secrete enzymes or other factors that negatively affect the bacteria or which alter the site. Preferably, growth of the non-pathogenic microorganism is not inhibited by a bacterium causing a colonization and/or infection. It is within the capacity of the skilled person to determine if growth of the non-pathogenic microorganism is inhibited by the bacteria causing colonization and/or infection, or vice versa. As an example, the growth of the non-pathogenic microorganism is not inhibited when the growth rate of said microorganism is the same in the presence and in the absence of the bacterium causing colonization and/or infection.

Specific examples of non-pathogenic microorganisms are provided herein. The skilled person will recognize appropriate ways to modify any particular disclosure herein such that it is applicable to additional genera, species, strains and isolates of non-pathogenic microorganisms. Non-pathogenic microorganisms which naturally exhibit desired growth and colonization behavior, and which are naturally resistant to the antibiotic used in the combination product, are preferably be used. Alternatively, non-pathogenic microorganisms can be manipulated, using conventional procedures, to enhance their ability to colonize a particular site, such as the skin or a mucus membrane, and/or to have appropriate antibiotic resistance. As a non-limiting example, a first method of manipulating a non-pathogenic microorganism involves repeatedly selecting for rapid colonization behavior on animal or human skin or mucosal layers (or in an appropriate *in vitro* model) in the presence of an antibiotic. Said microorganism may already be resistant to the antibiotic (e.g., naturally resistant or as a result of genetic engineering) or resistance may furthermore be selected for, e.g., by exposure to an antibiotic such that genetic modifications, such as mutations, that arise in the microorganism and that allow it to survive in presence of the antibiotic are maintained. As a particular example, a wild-type non-pathogenic microorganism may be applied to a mucosal surface or culture media in the presence of an antibiotic and be subjected to one or more cycles of isolation. Ultimately, a non-pathogenic microorganism with enhanced colonizing ability and/or that is resistant to the antibiotic may be obtained. In particular, non-pathogenic microorganisms known to be efficient colonizers by virtue of their use as "probiotics," which are live microorganisms which when administered in adequate amounts confer health benefits on the host, may be used. Typically, probiotic bacteria have demonstrated safety in human use, survival in the intestine, adhesion to mucosa, and at least temporary colonization of the gut or other skin or mucosal surface(s).

A particular example of a suitable non-pathogenic microorganism is a microorganism of the *Corynebacterium* genus. Indeed, *Corynebacterium sp.* naturally colonizes the nares and/or vaginal mucosa of healthy individuals. Furthermore, *Corynebacterium* species, such as *C. aurimucosum, C. philbarense,* and *C. afermentans,* are naturally resistant to fosfomycin. Preferably, the non-pathogenic microorganism belongs to the *Corynebacterium* genus, more preferably *C. aurimucosum, C. philbarense,* or *C. afermentans,* even more preferably *C. aurimucosum* CIP 102616, *C. philbarense* CRBIP 17.59, or *C. afermentans subsp. lipophilum* (e.g., *C. afermentans subsp. lipophilum* CIP 103500T). Preferably, said microorganism is resistant to fosfomycin or rifampicin.

A further example of a suitable non-pathogenic microorganism is a microorganism of the *Staphylococcus* genus, preferably *S. epidermidis,* which naturally colonizes the nares. Preferably, said microorganism is resistant to rifampicin.

The combination product comprises a sufficient quantity of the at least one non-pathogenic microorganism as to prevent emergence of resistance when administered simultaneously, separately, or sequentially with an antibiotic. Preferably, the combination product comprises at least 10³, 2.5x10³, 10⁴, 10⁵, 10⁶, 10⁷, 10⁸, 10⁹, 10¹⁰, 10¹¹, or 10¹² colony forming units (CFUs) of the non-pathogenic microorganism per dose. Preferably, the combination product of the present invention comprises from 10³ to 10¹² more preferably from 2.5x10³ to 10⁹ CFUs of the non-pathogenic microorganism per dose. The term "colony forming unit" or "CFU" is defined herein as a unit used to indicate the number of viable bacteria in a sample, wherein one colony forming unit corresponds to one viable bacterial cell.

In a preferred embodiment, the combination product of the invention further comprises a pharmaceutically acceptable excipient. When each product of the composition is provided in a separate container, each product may further comprise one or more pharmaceutically acceptable excipient(s). The term "pharmaceutically acceptable excipient" as used herein refers to a component, or combination of components, that is compatible with the combination product, does not generate unwanted side effects in the patient, and that is generally considered to be nontoxic. A pharmaceutically acceptable excipient is most commonly implicated in facilitating administration of the composition, increasing product shelf-life or efficacy, or improving the solubility or stability of one or both products of the composition. The pharmaceutically acceptable excipient is generally considered to be pharmacologically inactive. However, in some cases, the excipient itself may have a therapeutic effect. Pharmaceutically acceptable excipients or carriers are well-known in the prior art and can easily be adapted by the skilled person based on the desired galenic formulation of the composition. The galenic formulation, method of administration, and dosage can further be determined based on widely-accepted criteria for adapting patient treatments, including their general health, age, weight, tolerance to treatment, etc., as necessary. The choice of said one or more excipients may furthermore depend on the desired route of administration.

According to a particular aspect, the combination product as provided herein is administered to a subject either locally or systemically, via a local, enteral, or parenteral route or via a combination to two of said routes. Preferably, the antibiotic comprised in the combination product is administered via a local, enteral, or parenteral route, more preferably via a topical, oral, or parental route, even more preferably via the topical or oral route, most preferably via the topical route. Preferably, the non-pathogenic microorganism comprised in the combination product is administered via a local or enteral route, more preferably via a topical or oral route. The "parenteral" route of administration may be an intraperitoneal, intravenous, intradermal, intramuscular, intradermic, subcutaneous, or intra-articular route. "Topical" routes include administration to the skin and/or mucus membranes. As indicated above, said products may be administered simultaneously (thus, via the same route) or separately or sequentially (thus, via the same or different routes). The topical route is particularly preferred when bacterial colonization and/or infection is present on the skin and/or mucus membranes to ensure direct, local delivery of the non-pathogenic bacteria and/or antibiotic. The oral route is particularly preferred when bacterial colonization and/or infection is present within the gastrointestinal tract. According to a preferred embodiment, the antibiotic is administered systemically while the non-pathogenic microorganism is administered locally. According to a particularly preferred embodiment, the antibiotic is administered via the parenteral or oral route while the non-pathogenic microorganism is administered via the topical route. According to a particularly preferred embodiment, both the antibiotic and the non-pathogenic microorganism are administered via the topical route (e.g., to the skin or mucus membranes).

Formulations of the antibiotic and/or non-pathogenic microorganism comprised in the combination product can thus notably include those suitable for topical (including to skin or mucus membranes), oral and/or parenteral administration. The formulations may conveniently be presented in unit dosage form. The amount of antibiotic and/or non-pathogenic microorganism which can be combined with a carrier material or excipient to produce a single dosage form may vary depending upon the subject and/or the particular mode of administration. The amount of antibiotic and/or non-pathogenic microorganism, which can be combined with a carrier material to produce a single dosage form, will generally be the amount of the product that produces the desired effect when administered in combination with the other product. Thus, the amount of antibiotic may in some cases be lower than when said antibiotic is administered alone.

In the context of the present invention, the pharmaceutically acceptable excipient may notably comprise one or more antioxidants, buffers, bulking substances, suspending agents, solubilizing agents, matrix forming additives, humectants, diluents, solvents, plasticizers, oily/emulsifying/aqueous bases, gelling agents, preservatives, tonicity adjusting agents, vehicles, and stabilizers. As a non-limiting example, the combination product may comprise at least one pharmaceutically acceptable excipient selected from water, glycerin, mineral oils (e.g. vaseline, paraffin), animal oils or fats (e.g. beeswax or wool fat), semi-solid hydrocarbons (oleaginous), or vegetable oils (e.g. coconut oil, mango butter, palm oil, soybean oil, olive oil, shea butter and cacao butter), glyceride, xanthan gum, lactose, dextran, mannitol, methylcellulose, hydroxypropyl methylcellulose, hydroxypropyl cellulose, hydroxyethyl cellulose, sodium carboxy methylcellulose, carboxypropyl methylcellulose, polyethelyne glycol, polypropylene glycol, hyaluronic acid, starch-based biodegradeable polymers, tragacanth, pectin, chitin and chitosan derivatives, carrageenan, guar gum, agar, alginate, gelatin, fibrin, albumin, phosphate buffered saline, Polycarbophil, hydrogenated palm oil, glyceride, Carbomer 934P, macrogol, methyl paraben, glyceryl polymethacrylate, cyanoacrylate, triethanolamine, sorbic acid, NaOH, Carbopols (e.g. Carbopol 974P), diazolidinyl urea, Poloxamer 184, dimethicone, cellulose gum, lactic acid, methyl paraben, propylparaben, polyvinyl pyrrolidone, polyvinyl pyrrolidone-vinyl acetate, polyvinyl alcohol, polyacrylic acid, polyacrylamide, homo- and copolymers of acrylic acid crosslinked with a polyalkenyl polyether, sodium hydroxide, sodium chloride, and potassium chloride, and the like.

For oral administration, the antibiotic and/or non-pathogenic microorganism will generally take the form of a tablet or capsule, or may be an aqueous or nonaqueous solution, suspension or syrup. Tablets and capsules are preferred oral administration forms. Tablets and capsules for oral use will generally include one or more commonly used carriers such as lactose and corn starch. Lubricating agents, such as magnesium stearate, are also typically added. When liquid suspensions are used, the active agent may be combined with emulsifying and suspending agents. If desired, flavoring, coloring and/or sweetening agents may be added. Other pharmaceutically acceptable carriers for incorporation into an oral formulation include preservatives, suspending agents, thickening agents, and the like.

Topical administration to mucosal surfaces can be obtained by formulating the antibiotic and/or non-pathogenic microorganism as an inhalable, as a spray, and the like (e.g., nasal spray, aerosol spray or pump spray and the like), as a solution, as a gel, etc. Nebulizer devices suitable for delivery of active ingredients to the nasal mucosa, trachea and bronchioli are well-known in the art.

The antibiotic and/or non-pathogenic microorganism may notably be topically administered to the skin and/or mucosal surface in the form of a patch, gel, cream, lotion, ointment, paste, film, salve, or foam. Advantageously, the viscosity or texture of the antibiotic and/or non-pathogenic microorganism is modulated to improve application or efficacy (e.g., contact time). As a particular example, the antibiotic and/or non-pathogenic microorganism may comprise a vehicle selected in the group comprising solutions, emulsions, microemulsions, oil-in-water emulsions, anhydrous lipids and oil-in-water emulsions, other types of emulsions in view of administration to one or more of the mucosal surfaces described above.

The antibiotic and/or non-pathogenic microorganism comprised in the combination product provided herein may be administered more specifically to the rectal or vaginal mucosa as a solution, enema, foam, or suppository. Preferred vehicles for rectal or vaginal delivery include hydrophilic and hydrophobic vehicles such as those commonly used in formulating emulsion or gel preparations (e.g., oil/water emulsion gel).

Preferably, the non-pathogenic microorganism and/or antibiotic is provided in the form of a gel, more particularly a bioadhesive or mucoadhesive gel. The term "gel" is defined herein as a dispersion of particles interpenetrated with a liquid to generate a semisolid material. The term "bioadhesive gel" is defined herein as a gel which adheres to a biological surface (e.g., stratified squamous epithelium such as the skin or anterior nares). The term "mucoadhesive gel" is defined herein as a gel which adheres to a mucosal coat or surface (e.g., mucus membrane).

In the context of a gel, the at least one pharmaceutically acceptable excipient is preferably a gelling agent selected from hydroxypropyl methylcellulose, hydroxyethyl cellulose, chitosan and derivatives thereof, for example chitin, carrageenan and derivatives thereof, alginate and derivatives thereof, pectin and derivatives thereof, homo- and copolymers of acrylic acid crosslinked with a polyalkenyl polyether, or mixtures thereof. If a tonicity adjusting agent is present, said tonicity adjusting agent is preferably selected from potassium chloride, mannitol, dextrose, glycerin, or sodium chloride. Preferably, the antibiotic and/or non-pathogenic microorganism comprised in the combination product comprises at least one gelling agent and at least one tonicity adjusting agent. Preferably, sodium chloride is present in the combination product at an isotonic concentration. If a vehicle is present, said vehicle is preferably selected from water, a water miscible solvent (e.g., glycerin) or a water immiscible solvent (e.g., vegetable oil). If a preservative is present, said preservative is preferably selected from benzyl alcohol, cresols, benzoic acid, phenol, parabens and sorbic acid. If a stabilizer is present, said stabilizer is preferably selected from surfactants, polymers, polyols, a poloxamer, albumin, gelatin, trehalose, proteins, sugars, polyvinylpyrrolidone, N-acetyl-tryptophan ("NAT")), caprylate (i.e., sodium caprylate), a polysorbate (i.e., P80), amino acids, and divalent metal cations such as zinc.

Preferably, the antibiotic and/or non-pathogenic microorganism is provided in the form of an ointment, paste, lotion, or foam. An "ointment" as used herein refers to a semi-solid vehicle composed of hydrophobic constituents. Ointments can take the form of non-hydrocarbon ointment. Ointments can be formulated to provide a non-greasy, cosmetically acceptable appearance. As used herein, a "paste" is an ointment with a high loading of insoluble solids (up to 50% by weight) that forms a structured particulate matrix. A "lotion" as used herein refers to a dermatological vehicle that is a pourable suspension of insoluble powder (e.g., antibiotic) in a liquid. A "foam" as used herein is a disperse system consisting of a three-dimensional network of films in air.

According to a further aspect, the non-pathogenic microorganism comprised in the combination product is lyophilized or freeze-dried. "Lyophilization" is a process well-known to the skilled person for the removal of water from frozen bacterial cultures by sublimation under reduced pressure. General methods are described in "Lyophilization: Introduction and Basic Principles" (Jennings, 1999). Said process includes the following steps: providing an aqueous formulation comprising viable bacteria, freezing, primary drying (sublimation), and secondary drying (desorption). Lyophilized bacteria are obtained from said process.

Preferably, the non-pathogenic microorganism is lyophilized in the presence of a lyoprotectant. The lyoprotectant may be intracellular or extracellular or a combination of both. Preferably, said lyoprotectant is selected from peptone, glycerol, lactose, gelatin, glucose, sucrose, trehalose, dextran, maltodextrin, adonitol, and sodium glutamate. Preferably, the non-pathogenic microorganism is lyophilized in presence of a matrix forming additive. Preferably, said matrix forming additive is mannitol or skim milk. Preferably, the lyophilized non-pathogenic microorganism is stored at refrigeration temperatures (e.g., +3 to +6°C) or at room temperature (e.g., between 20 and 25°C).

In cases where the non-pathogenic microorganism is comprised of two or more species or strains of microorganism, each non-pathogenic microorganism is preferably lyophilized separately. Preferably, each lyophilized non-pathogenic microorganism is reconstituted separately with at least one pharmaceutically acceptable excipient just before use. Reconstituted non-pathogenic microorganisms may then be administered individually or admixed prior to administration. According to a second preferred embodiment, the at least two non-pathogenic microorganisms are admixed together following lyophilization. In this preferred embodiment, lyophilized non-pathogenic microorganisms are reconstituted together with at least one pharmaceutically acceptable excipient just before use. According to a third preferred embodiment, all non-pathogenic microorganisms are admixed together prior to lyophilization. In this preferred embodiment, the mixture may be reconstituted with at least one pharmaceutically acceptable excipient just before use.

The "reconstitution" of the lyophilized non-pathogenic microorganism is defined herein as placing the non-pathogenic microorganism in contact with a specific amount of at least one pharmaceutically acceptable excipient (e.g., a liquid or gel), such that said excipient hydrates the lyophilizate. Preferably, the mixture is agitated or stirred to ensure complete hydration. Preferably, reconstitution is performed within the range of 17°C to 37°C, more preferably at or above room temperature (20°C to 25°C), even more preferably at room temperature.

In one aspect, when the combination product comprises at least two non-pathogenic microorganisms and said microorganisms are administered separately or sequentially to the antibiotic, said at least two non-pathogenic microorganisms may also be administered separately, one after the other. Alternatively, separately reconstituted non-pathogenic microorganisms may be admixed prior to administration for simultaneous application.

Preferably, the composition is reconstituted immediately before use. If the reconstituted non-pathogenic microorganism is not administered immediately, it is preferably refrigerated until administration. If the reconstituted non-pathogenic microorganism is not administered immediately and is not refrigerated, it is preferably administered the same day as reconstitution. The skilled person can easily select appropriate reconstitution and storage conditions according to their general knowledge. Alternatively, viable non-pathogenic microorganism(s) may be stored at temperatures comprised between about +4°C and +8°C (e.g., for several days), at temperatures comprised between about -25°C and -40°C (e.g., for several weeks), or at temperatures comprised between about -72°C and -85°C (e.g., for several years). Lyophilized non-pathogenic microorganism(s) may also be stored at temperatures comprised between +4°C and +8°C for several days following reconstitution. Appropriate storage conditions at each of these temperature ranges are well-known to the person skilled in the art.

A further aspect of the present invention is a method of preventing emergence of antibiotic resistance when treating a colonization and/or an infection caused by bacteria, comprising administering an effective amount of an antibiotic simultaneously, sequentially or separately to the administration of an effective amount of at least one non-pathogenic microorganism to a subject in need thereof, wherein said bacteria is susceptible to said antibiotic and said non-pathogenic microorganism is resistant to said antibiotic. The method of preventing emergence comprises any of the aspects described herein.

The "patient" or "subject" may be as any human individual, regardless of their age. Specifically, the subject may be an adult or child. The term "adult" refers herein to an individual of at least 16 years of age. The term "child" comprises infants from 0-1 years of age and children from 1-8 years of age, 8-12 years of age, and 12-16 years of age. The term "child" further comprises neonatal infants from birth to 28 days of age and post-neonatal infants from 28 to 364 days of age. The composition may be administered to an adult or a child, including a neonatal infant.

The terms "treat," "treatment," "treating," and the like as used herein refers to a process in which bacterial colonization is reduced or, preferably, completely eradicated and/or in which the clinical symptoms of a bacterial infection are improved or, preferably, completely eliminated. Bacterial colonization is reduced when the number of undesirable bacteria present during and preferably following treatment is reduced when compared to the number of bacteria present prior to treatment with the combination product. The symptoms of a bacterial infection are improved when the number and/or severity of clinical symptoms is reduced during and, preferably, following, treatment when compared to that present prior to treatment with the combination product. "Treating" notably comprises preventing a bacterial infection from developing following bacterial colonization at the same site or at a different site.

The present invention also relates to the use of the combination product according to any of the embodiments described herein in the prevention of emergence of antibiotic resistance when treating a colonization and/or an infection caused by bacteria, wherein said bacteria is susceptible to said antibiotic and said non-pathogenic microorganism is resistant to said antibiotic.

A further aspect of the present invention is a combination product for use in the prevention of emergence of antibiotic resistance when treating a colonization and/or an infection caused by a bacterium, comprising at least one non-pathogenic microorganism, which is used in combination with an antibiotic, wherein said bacterium is susceptible to said antibiotic and said non-pathogenic microorganism is resistant to said antibiotic. A combination product for use as described above wherein the at least one non-pathogenic microorganism is simultaneously, sequentially, or separately administered with the antibiotic is also provided herein. A further aspect of the present invention is a combination product for use in the prevention of emergence of antibiotic resistance when treating a colonization and/or an infection caused by a bacterium comprising an antibiotic, which is used in combination with at least one non-pathogenic microorganism, wherein said bacteria is susceptible to said antibiotic and said non-pathogenic microorganism is resistant to said antibiotic. A combination product for use as described above wherein the antibiotic is simultaneously, sequentially, or separately administered with the at least one non-pathogenic microorganism is also provided herein. The method of treating comprises any of the aspects described herein.

According to a further aspect, a kit comprising the combination product as provided herein and instructions for administering the combination product described herein to a subject is also provided herein.

### FIGURE LEGENDS

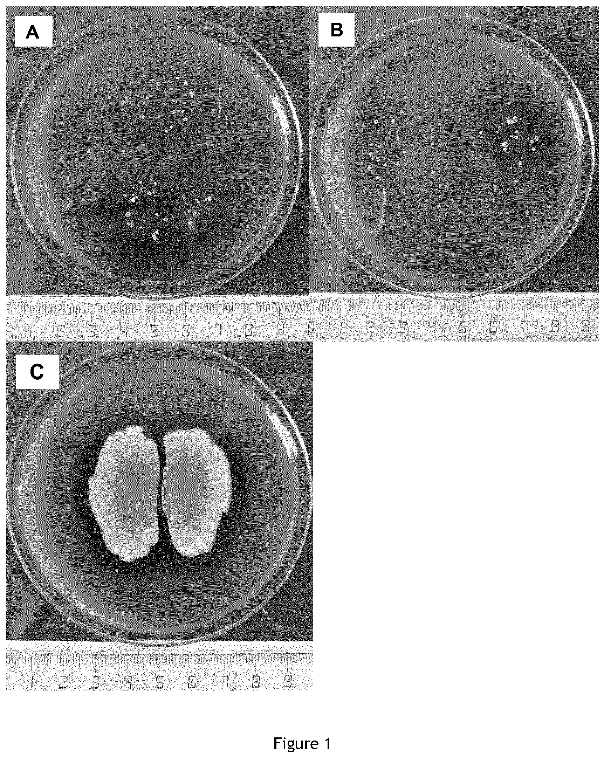

**Figure 1****: Development of fosfomycin-resistant mutants when *S. aureus* is grown on fosfomycin-containing solid media**
   **A** and **B.** Colonies of fosfomycin-resistant mutants are visible after 48h of incubation of *S. aureus* ATCC 29213 (A) or NCTC 12493 (B). Two drops each containing ≈10⁷ CFUs were spread out on blood agar plates supplemented with 50 µg/mL of fosfomycin and incubated at 35+/-2°C. Note that colonies exhibit various morphological aspects with both strains. **C.** Growth of *S. aureus* ATCC 29213 after 48h of incubation on blood agar plates without fosfomycin. A similar aspect was observed with *S. aureus* NCTC 12493 (not shown).

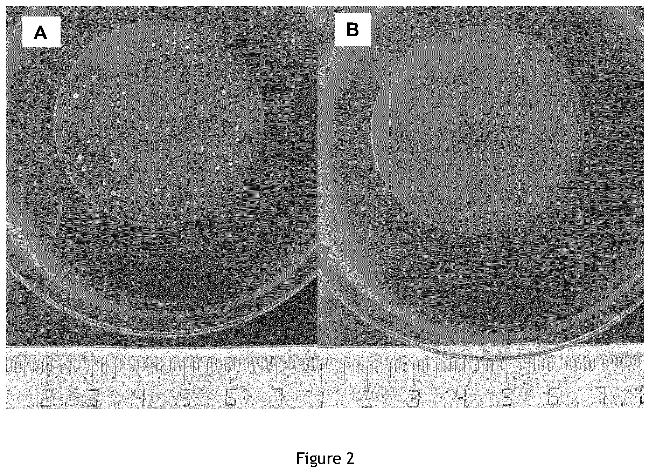

**Figure 2****: Fosfomycin-resistant colonies do not develop when *Corynebacterium aurimucosum* is co-cultured with *S*. *aureus* on fosfomycin-containing solid media** Two drops each containing ≈10⁷ CFUs of *S. aureus* ATCC 29213 were spread on a filter placed on top of a blood agar plate not previously inoculated **(A)** or previously inoculated **(B)** with ≈10⁷ CFUs of *C. aurimucosum* CIP 102616; plates were then incubated at 35+/-2°C for 48h. As shown, colonies of fosfomycin-resistant mutants do not develop when *C. aurimucosum* is co-cultured with *S. aureus.*

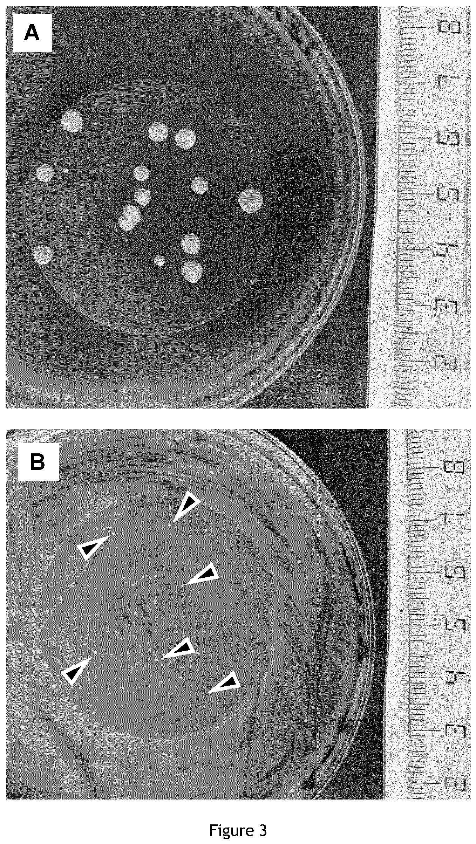

**Figure 3****: Co-culturing a *C*. *aurimucosum* Rif^{R} mutant with S. *aureus* in the presence of rifampicin strongly inhibits the development of S. *aureus* rifampicin-resistant colonies**
   A drop containing ≈10⁷ CFUs of *S*. *aureus* ATCC 29213 was spread out on a filter placed on top of a blood agar plate not previously inoculated (negative control) (A) or previously inoculated **(B)** with ≈10⁷ CFUs of *C. aurimucosum* CIP 102616 Rif^{R}; plates were then incubated at 35+/-2°C for 72 hours. As shown, the growth of S. *aureus* rifampicin-resistant colonies is strongly inhibited in presence of rifampicin and rifampicin resistant *C. aurimucosum.*

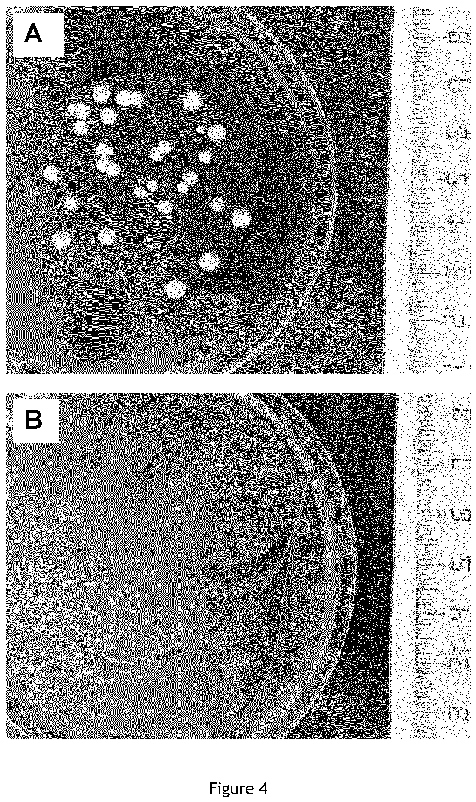

**Figure 4****: Co-culturing a *Staphylococcus epidermidis* Rif^{R} mutant with *S. aureus* in the presence of rifampicin strongly inhibits the development of *S. aureus* rifampicin-resistant mutants**
   A drop containing ≈10⁷ CFUs of *S. aureus* ATCC 29213 was spread out on a filter placed on top of a blood agar plate not previously inoculated (negative control) (A) or previously inoculated (B) with ≈10⁷ CFUs of *S. epidermidis* ATCC 12228 Rif^{R}; plates were then incubated at 35+/-2°C for 72 hours. As shown, the growth of *S. aureus* rifampicin-resistant colonies is strongly inhibited in presence of rifampicin and rifampicin resistant *S. epidermidis.*

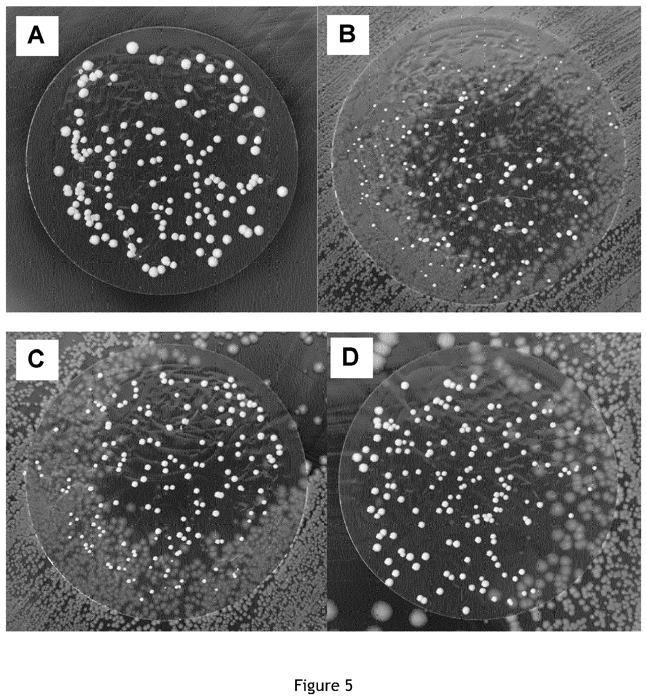

**Figure 5****: *Escherichia coli* does not prevent emergence of antibiotic-resistant *S. aureus***
   A drop containing ≈10⁷ CFUs of *S. aureus* ATCC 29213 was spread out on a filter placed on top of a blood agar plate not previously inoculated (negative control) **(A)** or previously inoculated with ≈10⁸ CFUs **(B),** ≈10⁷ CFUs **(C),** or ≈10⁶ CFUs **(D)** of *E. coli* ATCC 25922; plates were then incubated at 35+/-2°C for 72 hours. As seen in panels B, C and D, *E. coli* grew poorly where *S. aureus* was deposited, and did not prevent emergence of antibiotic-resistant *S. aureus.*

### EXAMPLES

The following examples are included to demonstrate preferred embodiments of the invention. All subject-matter set forth or shown in the following examples and accompanying drawings is to be interpreted as illustrative and not in a limiting sense. The following examples include any alternatives, equivalents, and modifications that may be determined by a person skilled in the art.

The representative bacterial strains used in these examples are detailed in Table I, below.

**Table I. Strains used.**

| **Strain** | **Source/origin/collection number** |
|---|---|
| *Staphylococcus aureus* NCTC 12493 | MRSA, DSM 27146 *S. aureus* Rosenbach 1884 |
| *Staphylococcus aureus* ATCC 29213 | MSSA, *S. aureus* subsp. *aureus* Rosenbach (strain Wichita) |
| *Staphylococcus epidermidis* ATCC 12228 | FDA strain PCI 1200 |
| *S. epidermidis* Rif^{R} mutant | Rifampicin-resistant mutant from *S. epidermidis* ATCC 12228; rifampicin MIC value > 256 µg/ mL^{1,2}; this study |
| *Corynebacterium aurimucosum* CIP 102616 | Human ear pus, Collection of the Pasteur Institute, Paris, France |
| *C. aurimucosum* Rif^{R} mutant | Rifampicin-resistant mutant from *C. aurimucosum* CIP 102616; rifampicin MIC value > 256 µg/mL^{1,2}; this study |
| *Corynebacterium afermentans subsp. lipophilum* CIP 103500T | Human blood, Strain Type, ATCC51404, JCM 10391, Strasbourg, France |
| *Corynebacterium philbarense* CRBIP 17.59 | Human eye, Biological Resource Center of Institut Pasteur, Paris, France |
| *Escherichia coli* ATCC 25922 | FDA Strain Seattle 1946 |

| | |
|---|---|
| ¹ Agar dilution method. ² Rifampicin resistance breakpoint: MIC value>16 µg/mL. Abbreviations: MRSA, methicillin-resistant *Staphylococcus aureus;* MSSA, methicillin-susceptible *Staphylococcus aureus;* MIC, minimum inhibitory concentration; Rif, rifampicin. | |

### Example 1: Fosfomycin-resistant mutants arise at high frequency when Staphylococcus aureus is exposed to fosfomycin.

Bacteria are known to easily develop fosfomycin resistance by mutation under fosfomycin treatment (Silver, 2017). *S. aureus* was used as a study model here. The frequency at which fosfomycin-resistant mutants appeared when *S. aureus* was grown *in vitro* on fosfomycin-containing agar plates was first determined. Two collection strains were tested: *S. aureus* ATCC 29213 (MSSA) and *S. aureus* NCTC 12493 (MRSA).

### Materials and Methods

Serially diluted suspensions of *S. aureus* ATCC 29213 and NCTC 12493 were prepared from a 24h-culture on Columbia 5% sheep blood agar at 35+/-2°C, and 20 µl drops of these suspensions were spread out on 90-mm diameter blood agar plates containing 50 µg/mL of fosfomycin, in order to obtain ≈10⁵, =10^{6,} ≈10⁷ and ≈10⁸ CFU per drop. Growth of fosfomycin resistant-mutants was determined by the presence of colonies visible with the naked eye after incubation for 24h, 48h and 72h at 35°C+/-2°C. The same series of experiments were performed by spreading out drops of bacterial suspensions onto a 47-mm diameter, 0.2 µm track-etched filter placed on top of the agar plate. Each set of experiments was repeated at least twice.

### Results

Fosfomycin resistant-mutants arose at a frequency of 1x to 4x10⁻⁶ when *S. aureus* ATCC 29213 or NCTC 12493 was grown on fosfomycin-containing agar plates. When ≈ 10⁷ *S. aureus* CFUs were inoculated, colonies of fosfomycin-resistant mutants were clearly seen with each strain after 48h of incubation on fosfomycin-containing agar plates (Figure 1). Similar results were obtained when *S. aureus* ATCC 29213 or NCTC 12493 were grown on 0.2 µm track-etched filters (data not shown).

### Conclusion

Colonies of fosfomycin-resistant mutants can be easily detected when ≈10⁷ *S. aureus* CFUs are grown on fosfomycin-containing solid media, or on 0.2 µm filters placed on top of fosfomycin-containing solid media.

### Example 2: Fosfomycin-resistant mutants do not develop when a non-pathogenic Corynebacterium species is co-cultured with S. aureus in presence of the antibiotic

*Corynebacterium* species are naturally resistant to fosfomycin (MIC values >256 µg/mL; fosfomycin resistance breakpoint >32 µg/mL). We studied whether a non-pathogenic *Corynebacterium* species could hamper the development of *S. aureus* fosfomycin-resistant mutants when co-cultured with *S. aureus.* Fosfomycin was used at a concentration of 50 µg/mL, which is in the range of concentrations obtained in patient tissues after the administration of intravenous fosfomycin at a dose of 5 g (Falagas, 2016).

### Materials and Methods

A 5 McFarland suspension of *Corynebacterium aurimucosum* CIP 102616 was prepared from a 48h-culture on Columbia 5% sheep blood agar at 35+/-2°C, and 300 µl of this suspension (≈10⁷ CFU) were inoculated by swabbing on a 90 mm diameter blood agar plate containing 50 µg/mL of fosfomycin; 300 µl of saline was inoculated as "negative control" (fosfomycin-containing agar plate not inoculated with *C. aurimucosum*). A 10 McFarland suspension of *S. aureus* (ATCC 29213 or NCTC 12493) was prepared as described in Example 1, and 20 µl drops of this suspension (≈10⁷ CFU) were spread out on a 47-mm diameter, 0.2 µm filter placed on top of the agar plate. Plates were incubated at 35+/-2°C, and the growth of fosfomycin-resistant mutants was determined as described in Example 1. Fosfomycin MIC values of bacteria forming fosfomycin-resistant colonies on "negative-control" plates were determined using the agar dilution method.

### Results

As previously found, colonies of fosfomycin-resistant mutants were detected when ≈10⁷ CFUs of *S. aureus* ATCC 29213 or NCTC 12493 were grown on a 0.2 µm filter placed on top of a fosfomycin-containing blood agar plate; in contrast, no such colonies appeared when *C. aurimucosum* was co-cultured with *S. aureus* (Figure 2). Fosfomycin MIC values of bacteria forming colonies on "negative-control" plates (fosfomycin-containing plates not inoculated with *C. aurimucosum*) were 32 µg/mL or higher, and thus confirmed to be fosfomycin-resistant mutants. Of note, of the fosfomycin-resistant mutant colonies, ≈50% had MIC values of 64 µg/mL or higher, and ≈30% had MIC values of 256 µg/mL or higher.

### Conclusion

Co-culturing *C. aurimucosum* with *S. aureus* in the presence of fosfomycin completely inhibits the development of fosfomycin-resistant *S. aureus* mutants, including mutants with high MIC values (MIC≥256 µg/mL). Thus, the emergence of fosfomycin-resistant *S. aureus* mutants is prevented.

### Example 3: The inoculation of low quantities of Corynebacterium aurimucosum are sufficient to abolish the development of S. aureus fosfomycin-resistant colonies

We previously showed that co-inoculating ≈10⁷ *C. aurimucosum* CFUs abolished the development of fosfomycin-resistant colonies when ≈10⁷ *S. aureus* CFUs were grown on fosfomycin-containing media. The minimal inoculum size of *C. aurimucosum* which was required was determined here in the same experimental conditions.

### Materials and Methods

Serial 1/5 dilutions were prepared from a 100 McFarland suspension of *C. aurimucosum* and 250 µl of each bacterial suspension was inoculated by swabbing on a 90 mm diameter blood agar plate containing 50 µg/mL of fosfomycin, in order to obtain ≈10⁹, ≈2x10⁸, ≈4x10⁷, ≈8x10⁶, ≈1.6x10⁶, ≈3.2x10⁵, ≈6.4x10⁴, ≈1.3x10⁴, ≈2.5x10³, ≈5x10², ≈10², and ≈20 CFU per plate. Materials and methods were otherwise as described in Example 2.

### Results

With both *S. aureus* ATCC 29213 and NCTC 12493, no colonies of *S. aureus* fosfomycin-resistant mutants could be detected when at least ≈2.5x10³ CFUs of *C. aurimucosum* were inoculated on plates.

### Conclusion

As illustrated here, low inoculums of *C. aurimucosum* are sufficient to totally abolish the development of *S. aureus* fosfomycin-resistant colonies in the presence of fosfomycin.

### Example 4: Corynebacterium philbarense prevents emergence of S. aureus fosfomycin-resistant mutants

We studied whether another non-pathogenic *Corynebacterium* species, *Corynebacterium philbarense,* had a similar effect to *C. aurimucosum* in preventing emergence of *S. aureus* fosfomycin-resistant mutants.

### Materials and Methods

A 5 McFarland suspension of *Corynebacterium philbarense* CRBIP 17.59 was prepared from a 48h-culture on Columbia 5% sheep blood agar at 35+/-2°C, and 300 µl of this suspension (≈10⁷ CFU) was inoculated by swabbing on a 90 mm diameter blood agar plate containing 50 µg/mL of fosfomycin. 300 µl of saline was inoculated as a "negative control" (fosfomycin-containing agar plate not inoculated with *C. philbarense*). Materials and methods were otherwise as described in Example 2.

### Results

Fosfomycin-resistant colonies developed when *S. aureus* ATCC 29213 or NCTC 12493 were cultured in presence of fosfomycin alone. As previously found with *C. aurimucosum,* co-culturing *C. philbarense* with *S. aureus* ATCC 29213 or NCTC 12493 in the presence of fosfomycin completely inhibited the development of fosfomycin-resistant *S. aureus* mutants (data not shown).

### Conclusion

As with *C. aurimucosum, C. philbarense* prevents the emergence of fosfomycin-resistant *S. aureus* mutants.

### Example 5: Corynebacterium afermentans subsp. lipophilum prevents development of S. aureus fosfomycin-resistant mutants

We studied whether another non-pathogenic *Corynebacterium* species, *Corynebacterium afermentans subsp. lipophilum*, had a similar effect to *C. aurimucosum* and *C. philbarense* in preventing the emergence of *S. aureus* fosfomycin-resistant mutants.

### Materials and Methods

A 5 McFarland suspension of *C. afermentans subsp. lipophilum* CIP 103500T was prepared from a 48h-culture on Columbia 5% sheep blood agar at 35+/-2°C, and 300 µl of this suspension (≈10⁷ CFU) was inoculated by swabbing on a 90 mm diameter blood agar plate containing 50 µg/mL of fosfomycin. 300 µl of saline was inoculated as negative control (fosfomycin-containing agar plate not inoculated with *C. afermentans subsp. lipophilum*). Materials and methods were otherwise as described in Example 2 using *S. aureus* ATCC 29213.

### Results

Fosfomycin-resistant colonies developed when *S. aureus* ATCC 29213 were cultured in presence of fosfomycin alone. Culturing *S. aureus* ATCC 29213 in the presence of both fosfomycin and *C. afermentans subsp. lipophilum* prevented the emergence of fosfomycin-resistant colonies of *S. aureus* ATCC 29213 (data not shown).

### Conclusion

As was observed for previously for *C. aurimucosum* and *C. philbarense, C. afermentans subsp. lipophilum* taken in combination with fosfomycin prevents emergence of fosfomycin-resistant *S. aureus* ATCC 29213.

### Example 6: A Rif^{R} C. aurimucosum mutant strongly inhibits the development of rifampicin-resistant S. aureus mutants

We previously showed that *C. aurimucosum* totally abolished the development of S. *aureus* fosfomycin-resistant mutants. As *S. aureus* also easily generates rifampicin-resistant mutants when exposed to the antibiotic (Aubry-Damon, et al. 1998), we studied here whether *C. aurimucosum* may also prevent the emergence of *S. aureus* resistant mutants, using a rifampicin-resistant mutant derived from *C. aurimucosum* CIP 102616.

### Materials and Methods

Spontaneous rifampicin-resistant colonies were obtained in one step by plating ≈10⁹ CFU of *C. aurimucosum* CIP 102616 on blood agar plates supplemented with 20 µg/mL of rifampicin. Five clones were re-isolated on rifampicin-containing plates, and one of them was selected *(C. aurimucosum* CIP 102616 Rif^{R} mutant).

A 5 McFarland suspension of CIP 102616 Rif^{R} was prepared from a 48h-culture on Columbia 5% sheep blood agar at 35+/-2°C, and 300 µl of this suspension (≈10⁷ CFU) was inoculated by swabbing on a 90 mm diameter blood agar plate containing 20 µg/mL of rifampicin; 300 µl of saline was inoculated as "negative control" (rifampicin-containing agar plate not inoculated with CIP 102616 Rif^{R}). Materials and methods were otherwise as described in Example 2, except that one 40 µl drop (≈10⁷ CFU) was spread out on each filter. Rifampicin MIC values of bacteria forming rifampicin-resistant colonies on "negative-control" plates were determined using the agar dilution method.

### Results

As shown on Figure 3A, large rifampicin-resistant colonies were seen when *S. aureus* ATCC 29213 was grown alone for 72 hours on rifampicin-containing agar plates. When co-culturing in the presence of *C. aurimucosum* Rif^{R}, rifampicin-resistant *S. aureus* colony size was significantly reduced, indicating that growth of resistant colonies was significantly inhibited in the combined presence of rifampicin and *C. aurimucosum* Rif^{R} (Figure 3B). Similar results were obtained with *S. aureus* NCTC 12493 (data not shown).

Rifampicin MIC values of bacteria forming colonies on "negative-control" plates (rifampicin-containing plates not inoculated with *C. aurimucosum*) were 16 µg/mL or higher, and thus confirmed to be rifampicin-resistant mutants. Of these, 100% had MIC values of 64 µg/mL or higher, ≈75% had MIC values of 128 µg/mL or higher, and ≈25% had MIC values of 256 µg/mL or higher.

### Conclusion

A *C. aurimucosum* Rif^{R} mutant strongly inhibited the growth of *S. aureus* rifampicin-resistant mutants, including mutants with MIC values of 256 µg/mL or higher. A *C. aurimucosum* Rif^{R} mutant can thus reduce the development of rifampicin-resistant mutants.

### Example 7: A Rif^{R} Staphylococcus epidermidis mutant strongly inhibits the development of rifampicin-resistant S. aureus mutants

We studied here whether a non-pathogenic microorganism of a different genus, *Staphylococcus,* may also prevent the emergence of *S. aureus* rifampicin-resistant mutants.

### Materials and Methods

A rifampicin-resistant mutant from *S. epidermidis* ATCC 12228 *(S. epidermidis* ATCC 12228 Rif^{R} mutant) was selected as described above in Example 6. A 5 McFarland suspension of this mutant was prepared from a 24h-culture on Columbia 5% sheep blood agar at 35+/-2°C, and 300 µl of this suspension (≈10⁷ CFU) was inoculated by swabbing on a 90 mm diameter blood agar plate containing 20 µg/mL of rifampicin; 300 µl of saline was inoculated as "negative control" (rifampicin-containing agar plate not inoculated with *S. epidermidis* Rif^{R}). Materials and methods were otherwise as described in Example 6.

### Results

Large rifampicin-resistant colonies were seen when *S. aureus* ATCC 29213 was grown for 48 hours on negative-control plates (rifampicin-containing agar plates not inoculated with *S. epidermidis* Rif^{R}) (Figure 4A). *S. aureus* colonies were as numerous but much smaller by co-culturing *S. epidermidis* Rif^{R} (Figure 4B). Similar results were obtained with *S. aureus* NCTC 12493 (data not shown).

### Conclusion

Like the *C. aurimucosum* Rif^{R} mutant evaluated previously (see Example 5), a *S. epidermidis* Rif^{R} mutant strongly inhibited the growth of *S. aureus* rifampicin-resistant mutants, and can thus reduce the development of rifampicin-resistant mutants.

### Example 8: Escherichia coli does not prevent the emergence of antibiotic resistance mutants of S. aureus

We studied here whether *Escherichia coli,* a Gram-negative enteric non-pathogenic microorganism may also prevent the emergence of *S. aureus* antibiotic-resistant mutants. This was studied using rifampicin.

### Materials and Methods

Serial 1:10 dilutions were prepared from a 5 McFarland suspension of *E. coli* ATCC 25922 previously cultured on Columbia 5% sheep blood agar at 35+/-2°C for 24 hours, and 300 µl of each bacterial suspension was inoculated by swabbing on a 90 mm diameter blood agar plate containing 20 µg/mL of rifampicin in order to obtain ≈10⁸, ≈ 10⁷, and ≈10°⁶ CFU per plate. 300 µl of saline was inoculated as a "negative control" (rifampicin-containing agar plate not inoculated with *E. coli* ATCC 25922). Materials and methods were otherwise as described in Example 2.

### Results

As shown in Figure 5 B, C, and D, the growth of *E. coli* was clearly inhibited by *S. aureus* NCTC 12493. It is noteworthy that *E. coli* was inhibited even when the ratio *E. coli:S. aureus* was 10:1 (see Figure 5B). Furthermore, the development of large *S. aureus* rifampicin-resistant colonies was not prevented by the presence of *E. coli.*

Similar results were obtained with *S. aureus* ATCC 29213 (data not shown).

### Conclusion

*E. coli* growth is inhibited by *S. aureus,* and is unable to prevent the emergence of antibiotic-resistant mutants of this pathogen.

Taken together, these results illustrate that various non-pathogenic microorganisms (e.g., of the *Corynebacterium* or *Staphylococcus* genera) that are resistant to a given antibiotic may be used in combination with said antibiotic in treating bacterial colonization and/or infection, such that emergence of resistance to the antibiotic in the treated bacteria can be prevented.

### References

Aubry-Damon, et al. 1998. Characterization of mutations in the rpoB gene that confer rifampin resistance in Staphylococcus aureus. Antimicrob Agents Chemother, 42(10):2590-2594.
Falagas et al. 2016. Fosfomycin. Clin Microbiol Rev, 29:321-347.
Jennings, 1999. Lyophilization: Introduction and Basic Principles. Interpharm/CRC Press, Denver.
Silver, 2017. Fosfomycin: mechanism and resistance. Cold Spring Harb Perspect Med, 7(2):a025262.
"United Nations meeting on antimicrobial resistance," Bull World Health Organ 2016; 94:638-639.

## Claims

1. Combination product comprising an antibiotic and at least one non-pathogenic microorganism for simultaneous, separate, or sequential use in preventing emergence of resistance to the antibiotic when treating a colonization and/or an infection caused by a bacterium that is susceptible to said antibiotic, wherein said at least one non-pathogenic microorganism is resistant to said antibiotic.

2. Combination product for use according to claim 1, wherein the non-pathogenic microorganism is naturally resistant to the antibiotic, is selected in the presence of an antibiotic, or is genetically modified to be resistant to the antibiotic.

3. Combination product for use according to claim 1 or 2, wherein resistance to the antibiotic is due to at least one nucleotide mutation in a gene or the introduction of a non-transferable gene.

4. Combination product for use according to any one of claims 1 to 3, wherein at least 10³ CFUs of the non-pathogenic microorganism is administered per dose, preferably 10³ to 10⁹ CFUs per dose.

5. The combination for use according any one of claims 1 to 4, wherein the non-pathogenic microorganism is administered on the same day as, but before, the antibiotic.

6. Combination product for use according to any one of claims 1 to 5, wherein the antibiotic is associated with the development of mutational resistance in bacteria when used alone.

7. Combination product for use according to any one of claims 1 to 6, wherein the antibiotic is selected from fosfomycin and rifamycins, such as rifampicin.

8. Combination product for use according to any one of claims 1 to 7, wherein the non-pathogenic microorganism is a bacterium or yeast, preferably a bacterium.

9. Combination product for use according to any one of claims 1 to 8, wherein the non-pathogenic microorganism belongs to the *Corynebacterium* or *Staphylococcus* genus.

10. Combination product for use according to claim 9, wherein the non-pathogenic microorganism is *Corynebacterium aurimucosum, Corynebacterium philbarense, Corynebacterium afermentans,* or *Staphylococcus epidermidis.*

11. Combination product for use according to any one of claims 1 to 10, wherein the non-pathogenic microorganism is *Corynebacterium aurimucosum, Corynebacterium philbarense,* or *Corynebacterium afermentans* and the antibiotic is fosfomycin or rifampicin.

12. Combination product for use according to any one of claims 1 to 11, further comprising at least one pharmaceutically acceptable excipient.

13. Combination product for use according to any one of claims 1 to 12, wherein the non-pathogenic microorganism is administered topically or orally, preferably topically.

14. Combination product for use according to any one of claims 1 to 13, wherein the antibiotic is administered topically, orally, or parenterally, preferably topically.
